# EUROPÄISCHE PATENTSCHRIFT

(11) **EP 3 562 415 B1**
(45) Veröffentlichungstag und Bekanntmachung des Hinweises auf die Patenterteilung: **26.02.2025**
(21) Anmeldenummer: 17828915.3
(22) Anmeldetag: 21.12.2017
(51) Int. Cl.: A61B 17/221, A61B 17/00

(54) **MEDIZINISCHES FANGNETZSCHLINGENINSTRUMENT**
MEDICAL NET-AND-LOOP-TYPE RETRIEVAL INSTRUMENT
INSTRUMENT MÉDICAL À BOUCLE À FILET DE RÉCUPÉRATION

(30) Priorität: 29.12.2016 DE 102016226295
(43) Veröffentlichungstag der Anmeldung: 06.11.2019
(73) Patentinhaber: EPflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner mbB
(86) Internationale Anmeldenummer: PCT/EP2017/084275
(87) Internationale Veröffentlichungsnummer: WO 2018/122136

(56) Entgegenhaltungen:
- EP-A1- 2 085 045
- EP-A2- 1 967 146
- US-A- 5 486 182
- US-A- 5 735 289
- US-A1- 2016 081 702

## Beschreibung

Die Erfindung bezieht sich auf ein medizinisches Fangnetzschlingeninstrument nach dem Oberbegriff der Ansprüche 1, 2 oder 3. Derartige Instrumente finden insbesondere in der medizinischen Endoskopietechnik Verwendung, z.B. als Polypektomieschlingeninstrument in der endoskopischen Polypektomie.

Es ist für medizinische Fangnetzschlingeninstrumente mit einem an einer distalen Schlinge gehaltenen Fangnetz bekannt, das Fangnetz auf die distale Schlinge quasi aufzufädeln, d.h. entsprechende Abschnitte des Netzgewebes sind lose um die Schlinge gewunden. Zur Verringerung von Verschiebebewegungen des Fangnetzes längs der Schlinge können Halteschlaufen eines zusätzlichen Haltefadens vorgesehen sein, die an der betreffenden Verbindungsstelle die Schlinge und ein angrenzendes Gewebestück des Fangnetzes umschließen. Die Offenlegungsschrift WO 2008/154406 A1 offenbart ein solches herkömmliches Fangnetzschlingeninstrument. Ein weiteres herkömmliches Fangnetzschlingeninstrument dieser Art ist in der Patentschrift EP 0 583 964 B1 für den Einschluss eines geformten inneren Organs oder von Gewebe während der endoskopischen Chirurgie in einem bioabsorbierbaren chirurgischen Beutel offenbart. Dort ist das Fangnetz von dem Beutel gebildet, der an seinem Umfangsrand einen Saum aufweist, durch den die Schlinge durchgefädelt ist. Diese herkömmlichen Fangnetzschlingeninstrumente sind in ihrer Herstellung relativ aufwendig, und im Gebrauch können unerwünschte Verschiebungen des Fangnetzes längs der Schlinge auftreten.

Medizinische Fangnetzschlingeninstrumente der eingangs genannten Art vermeiden solche Fangnetzverschiebungen längs der Schlinge, indem das Fangnetz mit einem Netzhalteabschnitt im Inneren des Querschnitts der Schlinge gehalten ist. Die Offenlegungsschrift EP 2 085 045 A1 offenbart ein solches Fangnetzschlingeninstrument, bei dem die Schlinge aus einem einzigen, längsgeschlitzten, als eine elektrochirurgische Elektrode ausgeführten Schlingenteil besteht und das Fangnetz aus einem schrumpffähigen Material gebildet und in den Längsschlitz der Schlinge eingefügt und dort längsbeweglich, aber gegen Herausgelangen aus dem Schlitz gesichert gehalten ist.

Die Offenlegungsschrift EP 1 967 146 A2 offenbart ein endoskopisches Kombi-Instrument zum Schneiden und Einfangen von Körpergewebe, wobei ein Fangteil des Instruments eine einteilige Schlinge rechteckförmigen Querschnitts beinhaltet, an der radial nach innen sukzessive aneinandergrenzend ein Fangbeutel, ein Schneidbeutel und ein Drahthaltestreifen fixiert sind, wobei der Drahthaltestreifen mit dem Schneidbeutel einen Aufnahmeraum für Schneiddrähte bildet.

Weitere herkömmliche medizinische Fangnetzschlingeninstrumente sind in der Offenlegungsschrift US 2016/0081702 A1 und den Patentschriften US 5,486,182 und US 5,735,298 offenbart.

Der Erfindung liegt als technisches Problem die Bereitstellung eines Fangnetzschlingeninstrumentes der eingangs genannten Art zugrunde, das die oben erwähnten Schwierigkeiten des Standes der Technik vermeidet oder jedenfalls vermindert und das sich insbesondere mit relativ geringem Aufwand herstellen lässt und bei dem das Fangnetz vergleichsweise sicher an der Schlinge gehalten wird.

Die Erfindung löst dieses Problem durch die Bereitstellung eines Fangnetzschlingeninstrumentes mit den Merkmalen des Anspruchs 1, 2 oder 3. Vorteilhafte Weiterbildungen der Erfindung sind in den Unteransprüchen angegeben.

Beim erfindungsgemäßen Fangnetzschlingeninstrument ist das Fangnetz von der Schlinge mit seinem Netzhalteabschnitt im Inneren des Querschnitts der Schlinge festgehalten. Dadurch lässt sich ein Verschieben des Fangnetzes längs der Schlinge problemlos vermeiden, und diese Halterung des Fangnetzes an der Schlinge kann fertigungstechnisch mit relativ geringem Aufwand realisiert werden. Zusätzliche Halteschlaufen zur Anbindung des Fangnetzes an die Schlinge und/oder zur Vermeidung unerwünschter Verschiebebewegungen des Fangnetzes entlang der Schlinge sind nicht erforderlich. Die Schlinge kann an ihrer Oberfläche und insbesondere an ihrem radial äußeren und/oder an ihrem radial inneren Rand ganz oder jedenfalls größtenteils frei vom Fangnetz gehalten werden und folglich dort einen glatten Verlauf aufweisen, ohne dass dieser glatte Verlauf durch dort befindliche Fangnetzteile oder Halteteile, wie Halteschlaufen und dgl., gestört wird.

Gemäß einem Aspekt der Erfindung beinhaltet die Schlinge mindestens zwei konforme Schlingenteile, d.h. die Schlingenteile besitzen eine übereinstimmende Schlingenform. Die konformen Schlingenteile liegen aneinander und sind in dieser aneinanderliegenden Lage miteinander zur Bildung der Schlinge verbunden, wobei sie zwischen sich einen Netzaufnahmespalt belassen. In diesem Netzaufnahmespalt ist das Fangnetz mit seinem Netzhalteabschnitt festgehalten. Die Schlingenteile können z.B. getrennt vorgefertigt und dann aneinander befestigt werden. Für die Schlingenteile können gleiche oder unterschiedliche Materialien verwendet werden, z.B. alle aus Metall oder alle aus Kunststoff oder mindestens eines aus Metall und mindestens ein anderes aus Kunststoff. Als Metall- bzw. Kunststoffmaterialien sind für die Schlinge vorliegend beliebige, hierfür an sich bekannte Materialien verwendbar, einschließlich superelastischer Materialien.

Gemäß einem speziellen Unteraspekt der Erfindung sind mindestens ein erstes und ein zweites der Schlingenteile mittels mindestens einer Klebestellenverbindung auf der vom Fangnetz abgewandten Seite und/oder mindestens einer Klemmstellenverbindung aneinander fixiert. Dies stellt vorteilhaft je nach Bedarf und Anwendungsfall realisierbare Verbindungen der Schlingenteile zu deren gegenseitiger Fixierung dar. In die Fixierung der Schlingenteile aneinander kann optional auch das Fangnetz einbezogen sein, das in diesem Fall zwischen mindestens zwei der Schlingenteile festgeklemmt und/oder mit mindestens einem der Schlingenteile verklebt und/oder verschweißt/verlötet und so im Netzaufnahmespalt festgehalten ist. Alternativ ist das Fangnetz mit einer von der Fixierung der Schlingenteile aneinander unabhängigen Verbindung im Netzaufnahmespalt festgehalten.

Gemäß einem weiteren speziellen Unteraspekt der Erfindung weist ein erstes Schlingenteil einen U-förmigen Querschnitt auf, und ein zweites Schlingenteil weist einen rechteckförmigen Querschnitt auf und wird vom ersten Schlingenteil U-förmig auf drei Seiten umgriffen.

In einer Ausgestaltung der Erfindung beinhaltet das Fixieren mindestens des ersten und des zweiten Schlingenteils aneinander die Verwendung mindestens einer Schweiß-/Lötstellenverbindung oder einer Klammer, die mindestens diese beiden Schlingenteile zusammenklemmend umgreift, entweder entlang der gesamten Länge der Schlinge oder wenigstens abschnittweise an einer oder mehreren beabstandeten Stellen entlang der Schlinge. Diese Fixierungsart kann fertigungstechnische Vorteile haben und optional gleichzeitig dazu dienen, das Fangnetz im Netzaufnahmespalt festgeklemmt zu halten.

Gemäß einem weiteren speziellen Unteraspekt der Erfindung beinhaltet die Schlinge ein äußeres und ein inneres Schlingenteil gleichen Querschnitts, wobei das äußere Schlingenteil das innere Schlingenteil umgibt und mit einem Innenumfang einem Außenumfang des inneren Schlingenteils zugewandt ist. Mit anderen Worten weist das innere Schlingenteil einen etwas geringeren Durchmesser bzw. eine etwas geringere Schlingenweite auf als das äußere Schlingenteil und wird von diesem koaxial umgeben. Das innere und das äußere Schlingenteil liegen somit koaxial in einer gleichen Schlingenebene. Dazwischen kann der Netzaufnahmespalt gebildet sein, in den das Fangnetz zum Beispiel in axialer Richtung, d.h. senkrecht zur Schlingenebene, eingefügt bzw. durchgeführt sein kann.

Gemäß einem weiteren Aspekt der Erfindung ist die Schlinge einteilig unter Einbettung des Netzhalteabschnitts des Fangnetzes in das Innere des Querschnitts der Schlinge gefertigt. Hierfür ist die Schlinge als gießtechnisches Bauteil aus Metall und/oder Kunststoff gefertigt, in welches das Fangnetz mit dem Netzhalteabschnitt eingegossen ist, speziell als einkomponentiges Kunststoffspritzgießteil oder als mehrkomponentiges Kunststoffspritzgießteil mit wenigstens einer ersten Schlingenkomponente aus Metall oder Kunststoff und einer daran angegossenen zweiten Schlingenkomponente vorzugsweise aus Kunststoff, wobei das Fangnetz mit seinem Netzhalteabschnitt zwischen den Schlingenkomponenten eingegossen gehalten ist. Auch diese Realisierung des erfindungsgemäßen Fangnetzschlingeninstruments gewährleistet eine sichere Halterung des Fangnetzes in Kombination mit einer funktionell vorteilhaften Gestaltung der Schlinge bei relativ geringem Fertigungsaufwand.

In einer Weiterbildung der Erfindung ist das Fangnetz mittels mindestens einer Schweiß-/Lötstellenverbindung an der Schlinge gehalten. Dies stellt eine vorteilhafte Verbindungsart für das Fangnetz mit der Schlinge dar, wobei es im Fall einer mehrteilig aufgebauten Schlinge je nach Bedarf und Anwendungsfall mit nur einem, mehreren oder allen Schlingenteilen der Schlinge verbunden sein kann.

In einer Weiterbildung der Erfindung ist das Fangnetz von einem Netzgewebebeutel gebildet, der mit einem Beutelrand an der Schlinge festgehalten ist, d.h. der Beutelrand bildet in diesem Fall den Netzhalteabschnitt des Fangnetzes. Diese Fangnetzausführung ist für entsprechende Anwendungen von Vorteil, und die Befestigung des Fangnetzes an der Schlinge erfolgt einfach durch das Festhalten des Beutelrandes im Inneren des Schlingenquerschnitts.

Gemäß einem weiteren Aspekt der Erfindung ist das Fangnetz von einem umfangseitig geschlossenen, zusammengelegten Netzgewebe-Schlauchstück gebildet, das sich in Umfangsrichtung durch das Innere des Querschnitts der Schlinge hindurch erstreckt. Das Fangnetz ist folglich in diesem Fall durch das Zusammenlegen des Netzgewebe-Schlauchstücks doppelwandig ausgeführt. Im Fall einer mehrteilig aufgebauten Schlinge kann zur Fertigung ein erstes der Schlingenteile in das Netzgewebe-Schlauchstück eingelegt und ein zweites der Schlingenteile außen um das Netzgewebe-Schlauchstück gelegt werden. Im Fall einer einteiligen Schlinge kann das Netzgewebe-Schlauchstück mit einem umfangsseitigen Teilabschnitt als Netzhalteabschnitt in den Querschnitt der Schlinge eingebettet sein. Die Verwendung des Netzgewebe-Schlauchstücks zur Bildung des Fangnetzes ermöglicht eine vorteilhaft einfache Fixierung des Fangnetzes an der Schlinge, indem es bereichsweise eingegossen wird bzw. indem mindestens das erste Schlingenteil in das Innere des Schlauchstücks eingelegt und mindestens das zweite Schlingenteil außen um das Schlauchstück gelegt wird. Da das Schlauchstück umfangseitig geschlossen ist, sind am Fangnetz umfangsseitig keine freien Gewebeenden vorhanden, die an der Schlinge gehalten werden müssten. Vielmehr erstreckt sich das Fangnetz als zusammengelegtes Schlauchstück einfach durch den Querschnitt der Schlinge hindurch und ist in dieser Lage durch die Schlinge gehalten bzw. fixiert. Diese Ausführungsvariante kann somit das Fangnetz in einer umlaufend geschlossenen Gewebeform ohne freie umfangsseitige Gewebeenden bereitstellen, allenfalls stirnseitig sind freie Gewebeenden vorhanden, wenn das Schlauchstück stirnseitig nicht geschlossen, sondern offen ist.

In einer Ausgestaltung der Erfindung ist mindestens ein erstes der Schlingenteile mit dem Netzhalteabschnitt des Fangnetzes und/oder mit mindestens einem zweiten der Schlingenteile mittels einer Klebestellenverbindung verbunden, die sich ausschließlich an einer oberen Seite der Schlinge befindet, wobei sich das Fangnetz nur an einer gegenüberliegenden unteren Seite in die Schlinge hinein erstreckt. Dies stellt eine fertigungstechnisch und funktionell vorteilhafte Realisierung des Fangnetzschlingeninstrumentes dar. Die Klebestellenverbindung kann längs der gesamten Schlinge durchgehend oder nur abschnittsweise angebracht sein.

Vorteilhafte Ausführungsformen der Erfindung sind in den Zeichnungen dargestellt und werden nachfolgend beschrieben. Hierbei zeigen:
- Fig. 1: eine Perspektivansicht eines distalen Endbereichs eines medizinischen Fangnetzschlingeninstrumentes mit Schlinge aus radial nebeneinanderliegenden Schlingenteilen und mit beutelartigem Fangnetz,
- Fig. 2: die Ansicht von Fig. 1 mit teilweise weggebrochenem äußerem Schlingenteil,
- Fig. 3: eine Draufsicht auf den distalen Instrumentenbereich von Fig. 1,
- Fig. 4: eine Perspektivansicht entsprechend Fig. 1 mit weggebrochener vorderer Schlingen-/Fangnetzhälfte,
- Fig. 5: eine schematische Schnittansicht längs einer Linie V-V in Fig. 4,
- Fig. 6: eine schematische Schnittansicht längs einer Linie VI-VI in Fig. 3 für eine Variante mit zusätzlicher Klebeverbindung,
- Fig. 7: die Schnittansicht von Fig. 6 für eine Ausführungsvariante mit zusätzlicher Klammer,
- Fig. 8: die Schnittansicht von Fig. 5 für eine weitere Ausführungsvariante mit zusätzlicher Klammer,
- Fig. 9: die Schnittansicht von Fig. 5 für eine Variante mit Schlingenteilen anderen Querschnitts,
- Fig. 10: die Schnittansicht von Fig. 5 für eine Variante mit axial ineinanderliegenden Schlingenteilen,
- Fig. 11: eine Perspektivansicht eines distalen Endbereichs eines weiteren Fangnetzschlingeninstruments in einem Vorfertigungstadium mit einem stirnseitig offenen Gewebeschlauchstück als Fangnetz und mit Schlinge aus äußerem und innerem Schlingenteil,
- Fig. 12: die Ansicht von Fig. 1 für das fertiggestellte Instrument von Fig. 11,
- Fig. 13: die Ansicht von Fig. 2 für das fertiggestellte Instrument von Fig. 11,
- Fig. 14: die Ansicht von Fig. 3 für das fertiggestellte Instrument von Fig. 11,
- Fig. 15: die Ansicht von Fig. 4 für das fertiggestellte Instrument von Fig. 11,
- Fig. 16: die Schnittansicht von Fig. 6 für das fertiggestellte Instrument von Fig. 11,
- Fig. 17: die Schnittansicht von Fig. 5 für eine Variante mit einkomponentig einteiligem Schlingenaufbau,
- Fig. 18: eine Ansicht zur Veranschaulichung eines Schrittes eines Verfahrens zur Herstellung des Instrumentes von Fig. 17,
- Fig. 19: die Ansicht von Fig. 18 zur Veranschaulichung eines weiteren Schrittes dieses Verfahrens,
- Fig. 20: die Schnittansicht von Fig. 5 für eine Variante mit zweikomponentig einteiligem Schlingenaufbau,
- Fig. 21: die Schnittansicht von Fig. 20 für eine Variante mit Gewebeschlauchstück für das Fangnetz,
- Fig. 22: die Schnittansicht von Fig. 20 für eine weitere Variante mit Gewebeschlauchstück für das Fangnetz,
- Fig. 23: die Schnittansicht von Fig. 20 für eine Variante mit um 90° gedrehter Schlingenanordnung und
- Fig. 24: die Schnittansicht von Fig. 5 für eine Variante mit Gewebeschlauchstück für das Fangnetz und dreiteiliger Schlinge.

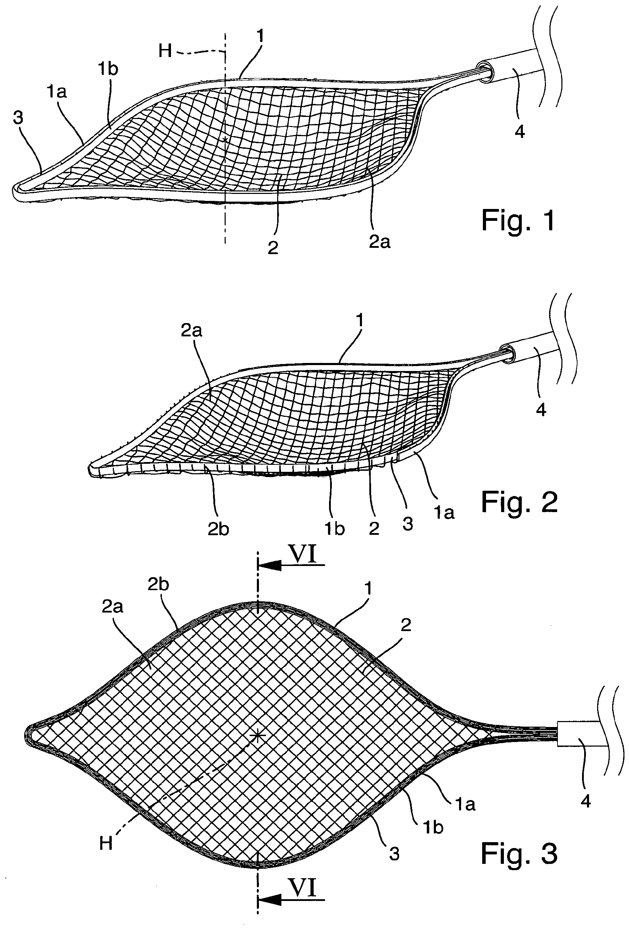

Die Fig. 1 bis 24 zeigen exemplarische Ausführungsbeispiele des erfindungsgemäßen medizinischen Fangnetzschlingeninstrument in seinem hier interessierenden, distalen Bereich. Das Instrument weist eine distale Schlinge 1 und ein an der Schlinge 1 gehaltenes Fangnetz 2 auf. Beim Ausführungsbeispiel der Fig. 1 bis 5 beinhaltet die Schlinge 1 mindestens zwei konforme Schlingenteile, d.h. zwei, drei, vier oder mehr konforme Schlingenteile, wobei sie im gezeigten Fall exemplarisch zwei Schlingenteile in Form eines ersten, hier äußeren Schlingenteils 1a und eines zweiten, hier inneren Schlingenteils 1b beinhaltet. Die mindestens zwei konformen Schlingenteile 1a, 1b sind unter Belassung eines Netzaufnahmespaltes 3 dazwischen aneinanderliegend miteinander verbunden, hier speziell radial nebeneinanderliegend. Das Fangnetz 2 ist mit einem Netzhalteabschnitt 2b im Netzaufnahmespalt 3 festgehalten und auf diese Weise mit dem Netzhalteabschnitt 2b von der Schlinge 1 im Inneren des Querschnitts der Schlinge 1 festgehalten, d.h. der im Netzaufnahmespalt 3 befindliche Teil des Fangnetzes bildet den Netzhalteabschnitt 2b. Die Schlingenteile 1a, 1b können jeweils z.B. einen langgestreckt ovalen oder im Wesentlichen rechteckigen Querschnitt besitzen, wie aus den Fig. 1 bis 5 ersichtlich, der Querschnitt der Schlinge 1 setzt sich demgemäß aus den Querschnitten der Schlingenteile 1a, 1b mit dem zwischenleigenden Netzaufnahmespalt 3 zusammen.

Die gezeigten Instrumente sind beispielsweise in der endoskopischen Polypektomietechnik als Polypektomieschlingeninstrument verwendbar. Dazu ist die distale Schlinge 1 in einer an sich bekannten Weise in eine schaftbildende Röhre 4 bzw. einen Schaftschlauch des Instruments einziehbar, wobei sich die Schlinge 1 entsprechend zusammenzieht bzw. zusammenfaltet. Dazu ist in ebenfalls an sich bekannter und daher hier nicht weiter zu zeigender und erörternder Weise die Schlinge 1 von einem durch die Schaftröhre 4 hindurch axial beweglich geführten Schlingendraht aus Metall oder Kunststoff gebildet. An einem proximalen Endbereich des Instruments sind der Schlingendraht und die Röhre 4 an eine Bedieneinheit gekoppelt, durch welche der Benutzer den Schlingendraht axial relativ zur Röhre 4 bewegen kann. Auf diese Weise kann der Benutzer die im Ausgangszustand vollständig in der Röhre 4 aufgenommene bzw. eingezogene Schlinge 1 aus der Röhre 4 herausbewegen, wodurch sich die Schlinge 1 von ihrem zusammengefalteten Zustand in der Röhre 4 in ihren in den Fig. 1 bis 4 gezeigten, aufgefalteten Zustand aufweitet. Das Herausbewegen der Schlinge 1 aus der Röhre 4 kann durch Vorschieben des Schlingendrahtes und/oder durch Zurückbewegen der Röhre 4 erfolgen. Der Schlingendraht kann z.B. aus einem superelastischen oder flexiblen Metall- oder Kunststoffmaterial bestehen, wie an sich bekannt. Dabei können die beiden Schlingenteile 1a, 1b je nach Bedarf aus dem gleichen Material oder aus unterschiedlichen Materialien bestehen, sei es aus zwei unterschiedlichen Kunststoffmaterialien oder aus zwei unterschiedlichen Metallmaterialien oder das eine aus einem Kunststoffmaterial und das andere aus einem Metallmaterial.

Das erste und das zweite Schlingenteil 1a, 1b sind vorzugsweise mittels mindestens einer Klemmstellenverbindung und/oder mindestens einer Klebestellenverbindung und/oder mindestens einer Schweiß-/Lötstellenverbindung aneinander fixiert. Beispielsweise kann dies durch mehrere Klebestellen- oder Schweiß-/Lötverbindungen realisiert sein, die im Abstand voneinander entlang der Schlinge 1 vorgesehen sind.

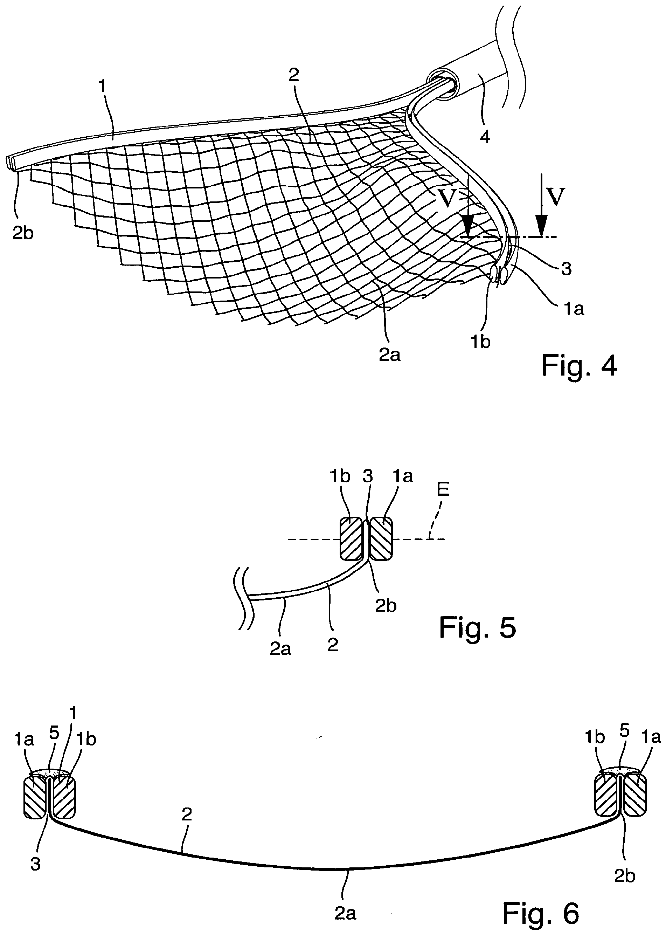

Wie aus den Fig. 1 bis 5 ersichtlich, umgibt das äußere Schlingenteil 1a konform, d.h. formgleich, das innere Schlingenteil 1b und ist mit einem Innenumfang einem Außenumfang des inneren Schlingenteils 1b zugewandt. Dazu weist das innere Schlingenteil 1b einen etwas geringeren Durchmesser bzw. eine etwas geringere Schlingenweite auf als das äußere Schlingenteil 1a und wird von dem äußeren Schlingenteil 1a koaxial umgeben. Das innere und das äußere Schlingenteil 1b, 1a liegen somit koaxial bezüglich einer Schlingenhochachse H, die als Ebenennormalenrichtung einer durch die Schlinge 1 aufgespannten bzw. definierten Schlingenebene E definiert ist und somit in der Draufsicht von Fig. 3 senkrecht zur Zeichenebene ist. Das Fangnetz 2 ist senkrecht zur Schlingenebene E und damit axial, d.h. in der Richtung der Schlingenhochachse H, in den Netzaufnahmespalt 3 eingefügt, in der Ansicht von Fig. 5 von unten.

Das Fangnetz 2 kann, wie exemplarisch zum Ausführungsbeispiel der Fig. 1 bis 5 gezeigt, von einem Netzgewebebeutel 2a gebildet sein, der mit einem Beutelrand als Netzhalteabschnitt 2b im Netzaufnahmespalt 3 festgehalten ist. Insbesondere kann dazu vorgesehen sein, das Fangnetz 2 mit seinem Beutelrand mittels mindestens einer Klemmstellenverbindung und/oder mindestens einer Klebestellenverbindung und/oder mindestens einer Schweiß-/Lötstellenverbindung an der Schlinge 1 zu halten. Beispielsweise kann das Fangnetz 2 mit seinem Beutelrand zwischen dem äußeren Schlingenteil 1a und dem inneren Schlingenteil 1b entlang der gesamten Länge der Schlinge 1 bzw. des Netzaufnahmespalts 3 oder wenigstens an mehreren beabstandeten Klemmstellen entlang der Schlinge 1 festgeklemmt gehalten sein. Die Klemmung kann dergestalt realisiert sein, dass allein dadurch das Fangnetz 2 sicher an der Schlinge 1 gehalten ist. Alternativ kann das Fangnetz 2 mit seinem Beutelrand zusätzlich am inneren oder am äußeren oder an beiden Schlingenteilen 1b, 1a durch eine oder mehrere Klebestellen und/oder eine oder mehrere Schweiß-/Lötstellen fixiert sein. Am Beutelrand vorliegende freie Enden des für das Fangnetz 2 verwendeten Gewebes können im Netzaufnahmespalt 3 aufgenommen werden, so dass sie nicht freiliegen oder anderweitig z.B. zu einem Saum umgearbeitet werden müssen. Das Netzgewebe des Fangnetzes 2 kann aus irgendeinem geeigneten Kunststoff- oder Metallmaterial bestehen, insbesondere einem der für Fangnetze von Polypektomieschlingeninstrumenten an sich bekannten Materialien.

Fig. 6 veranschaulicht eine Ausführungsvariante der in den Fig. 1 bis 5 gezeigten Ausführungsform, wobei als einziger Unterschied auf der in Fig. 6 oberen Seite der Schlinge 1 ein Klebematerial zur Bildung einer oder mehrerer Klebestellenverbindungen 5 aufgebracht ist. Hierbei kann es sich um eine längs der Schlinge 1 durchgehende Klebestellenverbindung oder um mehrere, voneinander beabstandet längs der Schlinge 1 aufgebrachte Klebestellenverbindungen handeln. Je nach Material der beiden Schlingenteile 1a, 1b und des Fangnetzes 2 stellt die Klebestellenverbindung 5 eine fixierende Verbindung zwischen dem Fangnetz 2 einerseits und dem ersten Schlingenteil 1a und/oder dem zweiten Schlingenteil 1b andererseits oder nur zwischen den beiden Schlingenteilen 1a, 1b her. Wenn die Klebestellenverbindung 5 die beiden Schlingenteile 1a, 1b aneinander fixiert, kann optional eine anderweitige Fixierung der beiden Schlingenteile 1a, 1b aneinander entfallen.

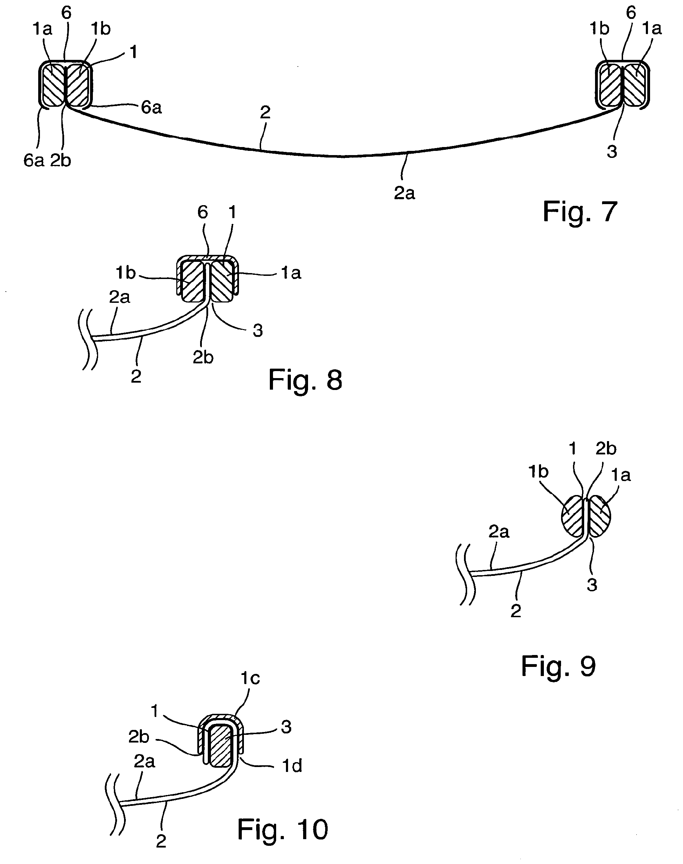

Fig. 7 zeigt eine weitere Ausführungsvariante des Instruments der Fig. 1 bis 5, wobei sich diese Variante darin unterscheidet, dass zusätzlich eine Klammer 6 vorgesehen ist, welche die Schlingenteile 1a, 1b wenigstens abschnittweise zusammenklemmend umgreift. Je nach Bedarf und Anwendungsfall kann sich die Klammer 6 durchgehend entlang der Länge der Schlinge 1 erstrecken oder eine oder mehrere einzelne Klammerteile beinhalten, die voneinander beabstandet an unterschiedlichen Stellen entlang der Länge der Schlinge 1 aufgesteckt sind. Im gezeigten Beispiel von Fig. 7 besitzt die Klammer einen im Wesentlichen U-förmigen Querschnitt mit leicht nach innen weisenden freien Klammerenden 6a. Die Klammer 6 ist in Fig. 7 von oben auf die Schlinge 1 aufgesetzt und hintergreift mit ihren einwärts gewandten freien Klammerenden 6a die Schlinge 1 bzw. deren beide Schlingenteile 1a, 1b. Die Klammer 6 lässt den Netzaufnahmespalt 3 auf derjenigen, in Fig. 7 unteren, Seite frei, an der das Fangnetz 2 mit seinem Beutelrand bzw. Netzhalteabschnitt 2b in den Netzaufnahmespalt 3 eingefügt ist.

Die Klammer 6 sorgt für eine sichere Klemmung und damit ein sicheres Fixieren der beiden Schlingenteile 1a, 1b aneinander. Gleichzeitig kann die Klammer 6 eine Klemmwirkung zum Festklemmen des Fangnetzes 2 mit seinem Beutelrand im Netzaufnahmespalt 3 zwischen den beiden Schlingenteilen 1a, 1b bereitstellen. Dadurch können optional anderweitige Verbindungs-/Fixiermittel zum Halten der beiden Schlingenteile 1a, 1b aneinander und/oder zum Festhalten des Fangnetzes 2 im Netzaufnahmespalt 3 entfallen.

Fig. 8 veranschaulicht eine Modifikation der Ausführungsvariante von Fig. 7 hinsichtlich der Klammer 6. Bei dieser modifizierten Ausführungsvariante endet die im Querschnitt U-förmige Klammer 6 an ihren freien Enden geradlinig, d.h. es fehlen die nach innen weisenden Klammerenden 6a der Ausführungsform von Fig. 7. Dementsprechend werden die Schlingenteile 1a, 1b von der Klammer 6 in der Ausführungsvariante von Fig. 8 nicht hintergriffen. Dennoch kann auch die Klammer 6 von Fig. 8 je nach Bedarf und Anwendungsfall eine ausreichende Klemmkraft zum Zusammenhalten der beiden Schlingenteile 1a, 1b und/oder zum festgeklemmten Halten des Fangnetzes 2 im Netzaufnahmespalt 3 zwischen den beiden Schlingenteilen 1a, 1b bereitstellen. Bei Bedarf kann die Klammer 6 durch zusätzliche Fixierungsmittel zum Fixieren der beiden Schlingenteile 1a, 1b aneinander und/oder des Fangnetzes 2 am ersten Schlingenteil 1a und/oder am zweiten Schlingenteil 1b unterstützt werden.

Fig. 9 zeigt eine Ausführungsvariante des Instruments der Fig. 1 bis 5 mit dem einzigen Unterschied, dass hier die beiden Schlingenteile 1a, 1b jeweils einen in etwa halbovalen Querschnitt mit einer im Wesentlichen planen und einer im Wesentlichen bogenförmigen Seite aufweisen. Dabei sind die beiden Schlingenteile 1a, 1b mit ihren planen Seiten einander zur Bildung des zwischen liegenden Netzaufnahmespaltes 3 zugewandt. Diese Ausführungsvariante resultiert in einem eher kreisrunden Querschnitt für die aus den beiden Schlingenteilen 1a, 1b gebildete Schlinge 1, während die Schlinge 1 im Ausführungsbeispiel der Fig. 1 bis 5 einen eher rechteckförmigen Querschnitt besitzt.

Fig. 10 zeigt als weiteres Ausführungsbeispiel der Erfindung ein medizinisches Fangnetzschlingeninstrument als Variante des Instruments der Fig. 1 bis 5 mit dem Unterschied, dass die Schlinge 1 aus einem ersten Schlingenteil 1c und einem zweiten Schlingenteil 1d gebildet ist, die nicht beide einen gleichen, zum Beispiel rechteckförmigen Querschnitt aufweisen, sondern von denen nur das zweite Schlingenteil 1d einen im Wesentlichen rechteckförmigen Querschnitt aufweist, während das erste Schlingenteil 1c einen U-förmigen Querschnitt aufweist und das zweite Schlingenteil 1d entsprechend U-förmig auf drei Seiten umgreift bzw. übergreift.

Dadurch ist bei dieser Ausführungsform der Netzaufnahmespalt 3 zwischen den beiden Schlingenteilen 1c, 1d entsprechend im Querschnitt U-förmig. In diesem hier U-förmigen Netzaufnahmespalt 3 ist das Fangnetz 2 festgehalten, wiederum dadurch, dass es mit seinem freien, offenen Beutelrand als Netzhalteabschnitt 2b des das Fangnetz 2 bildenden Beutels 2a in dem Netzaufnahmespalt 3 aufgenommen ist. Dabei liegt der Beutelrand über die volle Länge des U-förmigen Querschnitts des Netzaufnahmespaltes 3 in diesem, so dass er an drei Seiten der beiden Schlingenteile 1c, 1d zwischen diesen im Netzaufnahmespalt 3 festgehalten wird. Dies ermöglicht bei gegebener Festklemmkraft zwischen den beiden Schlingenteilen 1c, 1d eine sehr hohe Haltekraft für das Fangnetz 2 an der Schlinge 1. Bei Bedarf kann dies weiter durch eine klammerartige Ausführung des ersten Schlingenteils 1c unterstützt werden, wozu dieses dann mit seinen beiden U-Seiten klemmend auf das zweite Schlingenteil 1d aufgesetzt wird. Zuvor wird der Netzgewebebeutel 2a des Fangnetzes 2 mit seinem Beutelrand U-förmig um das zweite Schlingenteil 1d herum gelegt, so dass er beim Aufsetzen des ersten Schlingenteils 1c im Netzaufnahmespalt 3 festgeklemmt gehalten wird. Je nach Bedarf und Anwendungsfall können dann weitere Fixierungsmittel zum Festlegen des Fangnetzes 2 an der Schlinge 1 bzw. der beiden Schlingenteile 1c, 1d aneinander entfallen oder zusätzlich vorgesehen sein.

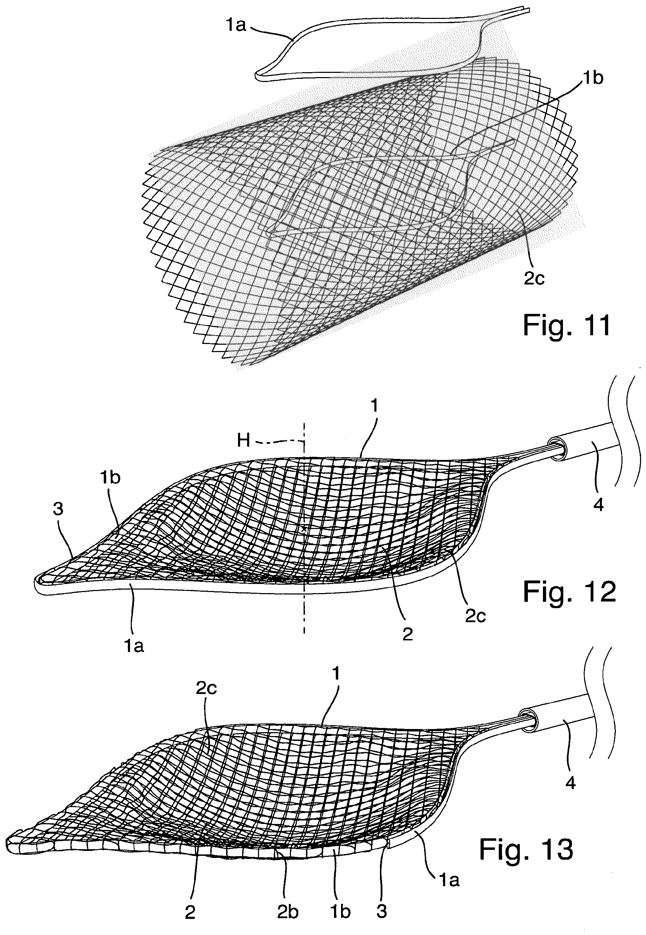

Die Fig. 11 bis 16 zeigen eine weitere Ausführungsform eines erfindungsgemäßen medizinischen Fangnetzschlingeninstrumentes, wobei hier das Fangnetz 2 von einem zusammengelegten Netzgewebe-Schlauchstück 2c gebildet ist. Im gezeigten Beispiel ist das Schlauchstück 2c umfangseitig geschlossen und an beiden Stirnseiten offen vorgefertigt bzw. vorbereitet, wie aus Fig. 11 zu erkennen. Für das Instrument der Fig. 11 bis 16 können ansonsten die gleichen Komponenten wie für das obige Ausführungsbeispiel der Fig. 1 bis 5 oder für das obige Ausführungsbeispiel von Fig. 10 verwendet werden, so dass insoweit auch auf dessen obige Beschreibung verwiesen werden kann. Dies gilt insbesondere für die Bildung der Schlinge 1 aus dem ersten Schlingenteil 1a bzw. 1c und dem zweiten Schlingenteil 1b bzw. 1d.

Zur Herstellung der fertigen Schlinge 1 mit dem Fangnetz 2 wird bei dieser Ausführungsvariante, wenn z.B. die Schlingenteile 1a und 1b des Ausführungsbeispiels der Fig. 1 bis 5 verwendet werden, das zweite, innere Schlingenteil 1b in das Innere des Netzgewebe-Schlauchstücks 2c eingelegt, wie aus Fig. 11 ersichtlich, und zwar von einer der beiden offenen Stirnseiten des Schlauchstücks 2c her. Dann wird das Schlauchstück 2c durch entsprechendes Zusammenlegen gegen das zweite Schlingenteil 1b angelegt und von einer Seite her zur Bildung einer gewünschten Netztasche durch das zweite Schlingenteil 1b durchgedrückt. Anschließend wird, wie ebenfalls in Fig. 11 veranschaulicht, das erste, äußere Schlingenteil 1a auf bzw. um das innere Schlingenteil 1b gelegt, bis das äußere Schlingenteil 1a seine das innere Schlingenteil 1b umgebende, koaxiale Lage einnimmt, wie sie aus den Fig. 12 bis 16 zu erkennen ist. Zuvor oder nach diesem Anbringen des äußeren Schlingenteils 1a kann das gegen das innere Schlingenteil 1b gelegte Netzgewebe-Schlauchstück 2c an seinen offenen Stirnseiten bei Bedarf geschlossen werden.

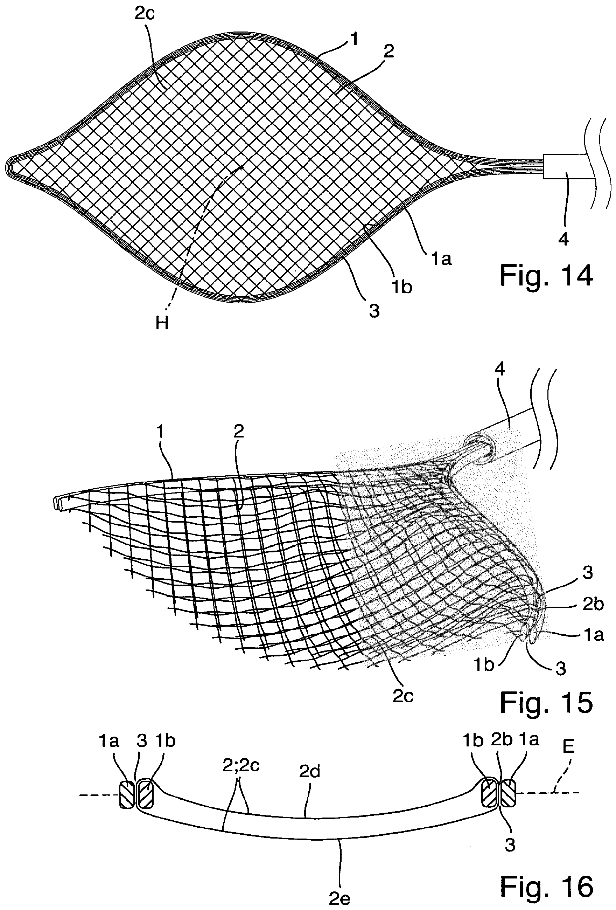

Die Fig. 12 bis 16 veranschaulichen das solchermaßen fertiggestellte Instrument in seinem hier interessierenden distalen Bereich entsprechend den Fig. 1 bis 5. Wie aus den Fig. 12 bis 16 zu erkennen, entsteht durch die Verwendung des Netzgewebe-Schlauchstücks 2c das Fangnetz 2 in einer doppelwandigen Taschen- bzw. Beutelform mit einer in den Fig. 12 bis 16 oberen Netzgewebelage 2d und unteren Netzgewebelage 2e. Da das Fangnetz 2 das innere Schlingenteil 1b in einer umfangseitig geschlossenen Form umgibt, wie insbesondere aus Fig. 16 zu erkennen, wird das Fangnetz 2 bei dieser Ausführungsform schon allein durch das innere Schlingenteil 1b gehalten. Das von außen koaxial gegen das innere Schlingenteil 1b anliegende äußere Schlingenteil 1a fixiert das Fangnetz 2 festhaltend in dieser Lage, indem es mit dem inneren Schlingenteil 1b wiederum den dazwischen liegenden Netzaufnahmespalt 3 bildet, in welchem das Fangnetz 2 mit seinem darin befindlichen Netzhalteabschnitt 2b festgehalten ist.

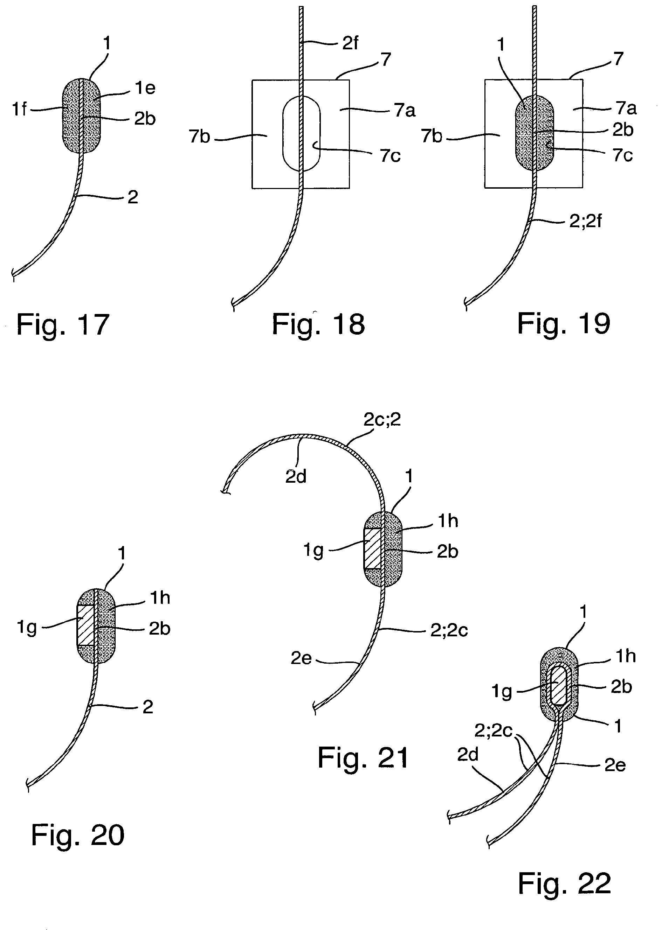

Bezüglich möglicher Fixierungen der beiden Schlingenteile 1a, 1b aneinander sowie optional des Fangnetzes 2 an einem oder beiden Schlingenteilen 1a, 1b sind für das Instrument der Fig. 11 bis 16 die gleichen Realisierungsvarianten möglich wie oben zu den Instrumenten der Fig. 1 bis 10 erläutert, worauf verwiesen werden kann. Das Instrument der Fig. 11 bis 16 lässt sich, wie erläutert, vergleichsweise einfach herstellen und ermöglicht ohne weiteren Zusatzaufwand die Bereitstellung des Fangnetzes 2 in einer doppelwandigen Ausführung und sehr sicheren Halterung an der Schlinge 1. Alternativ zu den Ausführungsbeispielen der Fig. 1 bis 16 mit zweiteiligem bzw. mehrteiligem Schlingenaufbau zeigen die Fig. 17 bis 22 exemplarische Ausführungsbeispiele eines ein- oder mehrkomponentig einteiligen Schlingenaufbaus.

Beim Ausführungsbeispiel von Fig. 17 ist die Schlinge 1 als ein einkomponentiges Kunststoffspritzgießbauteil gefertigt, in dessen Querschnitt das Fangnetz 2 mit seinem Netzhalteabschnitt 2b eingegossen ist. Die Schlinge 1 besteht in diesem Fall aus zwei sich bezüglich des eingegossenen Fangnetzes 2 gegenüberliegenden Schlingenhälften 1e, 1f, wobei beispielsweise die Schlingenhälfte 1e dem äußeren Schlingenteil 1a und die Schlingenhälfte 1f dem inneren Schlingenteil 1b des Ausführungsbeispiels der Fig. 1 bis 5 entsprechen kann. Das Fangnetz 2 kann z.B. wiederum beutel- bzw. taschenförmig nach Art des Netzgewebebeutels 2a des Ausführungsbeispiels der Fig. 1 bis 5 gebildet sein. Durch das Eingießen seines Netzhalteabschnitts 2b bzw. Beutelrandes in das Innere des hier z.B. oval gezeigten Querschnitts der Schlinge 1 ist das Fangnetz 2 ohne weitere Fixierungsmaßnahmen sicher an der Schlinge 1 gehalten.

Die Herstellung dieser Instrumentenvariante kann in Kunststoffspritzgießtechnik z.B. dergestalt erfolgen, dass zunächst ein Fangnetzvorläufer 2f in eine Spritzgießform 7 eingebracht wird, die in üblicher Weise mittels zweier Gießhälften 7a, 7b einen Gießformhohlraum 7c bereitstellt, wie in Fig. 18 schematisch veranschaulicht. Anschließend wird, wie in Fig. 19 schematisch veranschaulicht, das Kunststoffmaterial für die Schlinge 1 in den Gießformhohlraum 7c eingespritzt, wodurch der darin befindliche Netzhalteabschnitt 2b des Fangnetzes 2 in den Querschnitt der gegossenen Schlinge 1 eingebettet wird. Nach Entnahme der gegossenen Schlinge 1 mit dem eingegossenen Fangnetz 2 aus der Gießform 7 kann ein überstehender Teil des Fangnetzes abgeschnitten werden, vorzugsweise bündig mit der Oberfläche der einkomponentig gegossenen Schlinge 1.

Fig. 20 zeigt als weitere Ausführungsvariante der Erfindung ein Instrument mit zweikomponentig einteiligem, gegossenem Schlingenaufbau. Bei diesem Aufbau besteht die Schlinge 1 aus einer ersten Schlingenkomponente 1g und einer daran z.B. in Kunststoffspritzgießtechnik angegossenen zweiten Schlingenkomponente 1h. Im gezeigten Beispiel besitzt die erste Schlingenkomponente 1g einen rechteckförmigen Querschnitt, während der übrige Querschnitt der wie im Ausführungsbeispiel von Fig. 17 im Querschnitt ovalen Schlinge 1 vom angegossenen Kunststoffmaterial der zweiten Schlingenkomponente 1h gebildet ist. Das Fangnetz 2 befindet sich mit seinem Netzhalteabschnitt 2b wiederum in etwa mittig im Inneren des Querschnitts der Schlinge 1, zu einem Teil zwischen den beiden Schlingenkomponenten 1g, 1h und zu einem übrigen Teil im Inneren der beidseitig angespritzten zweiten Schlingenkomponente 1h. Wie im Ausführungsbeispiel von Fig. 17 kann auch hier jeweils eine Querschnittseite der Schlinge 1 auf der einen bzw. der anderen Seite des Fangnetzes 2 bzw. dessen Netzhalteabschnitts 2b dem radial äußeren Schlingenteil 1a bzw. dem radial inneren Schlingenteil 1b des Ausführungsbeispiels der Fig. 1 bis 5 entsprechen. Die erste Schlingenkomponente 1g kann z.B. aus einem superelastischen Metallmaterial bestehen, wie einer Nickel-Titan-Legierung. Zur Herstellung des Instruments kann die erste Schlingenkomponente 1g zusammen mit dem Netzhalteabschnitt 2b des Fangnetzes 2 in eine Kunststoffspritzgießform entsprechend der Gießform 7 von Fig. 18 eingelegt werden, wonach das Kunststoffmaterial für die zweite Schlingenkomponente 1h angespritzt wird.

Fig. 21 zeigt eine Ausführungsvariante der Erfindung, die derjenigen von Fig. 20 mit der Modifikation entspricht, dass als Ausgangsprodukt für das Fangnetz 2 kein Netzbeutel wie im Beispiel der Fig. 1 bis 5, sondern das Schlauchstück 2c entsprechend dem Ausführungsbeispiel der Fig. 11 bis 16 verwendet ist. Zur Herstellung kann in diesem Fall das Schlauchstück 2c mit einem Umfangsabschnitt als dem Netzhalteabschnitt 2b in eine Kunststoffspritzgießform entsprechend der Gießform 7 von Fig. 18 eingelegt werden, beispielsweise zusammen mit der ersten Schlingenkomponente 1g. In einer alternativen Ausführung mit einkomponentig einteiligem Schlingenaufbau wie beim Ausführungsbeispiel der Fig. 17 bis 19 wird nur das Schlauchstück 2c in den Gießformhohlraum eingelegt und mit dem Material der dann einkomponentigen Schlinge 1 im Netzhalteabschnitt 2b umspritzt.

Fig. 22 zeigt eine weitere Ausführungsvariante als Modifikation des Ausführungsbeispiels von Fig. 21. Bei der Ausführungsvariante von Fig. 22 mit zweikomponentig einteiligem Schlingenaufbau besteht die Schlinge aus einem Schlingenkern als der ersten Schlingenkomponente 1g und einer Umhüllung als der zweiten Schlingenkomponente 1h aus einem Kunststoffmaterial, welches die kernbildende erste Schlingenkomponente 1g im Querschnitt vollständig umgibt, d.h. das Material der kernbildenden ersten Schlingenkomponente 1g ist in diesem Fall vom Material der hüllenbildenden zweiten Schlingenkomponente 1h umgossen, d.h. in dieses eingegossen. Zwischen den beiden Schlingenkomponenten 1g, 1h befindet sich der Netzhalteabschnitt 2b des wiederum zweilagig mit den Netzlagen 2d und 2e aus dem Schlauchstück 2c gebildeten Fangnetzes 2. Dabei umschlingt im gezeigten Beispiel der Netzhalteabschnitt 2b die kernbildende Schlingenkomponente 1g umfangsseitig vollständig, so dass beide Netzlagen 2d, 2e aneinanderliegend an einer gleichen Seite aus der Schlinge 1 herausgeführt sind, in der Querschnittansicht von Fig. 22 an der unteren Schmalseite der Schlinge 1. Bei dieser Ausführungsvariante bleibt die Schlinge 1 somit umfangsseitig bis auf die Austrittsstelle des Fangnetzes 2 an der Schlingenschmalseite vollständig frei vom Fangnetz 2 und kann insoweit z.B. einen vollständig glatten Oberflächenverlauf entsprechend der gegossenen hüllenbildenden Schlingenkomponente 1h besitzen. Das Fangnetz 2 ist durch das Umschließen der kernbildenden Schlingenkomponente 1g und das Umgießen durch das Material der hüllenbildenden Schlingenkomponente 1h sehr sicher an der Schlinge 1 gehalten, ohne dass es anderweitiger Fixierungsmaßnahmen bedarf.

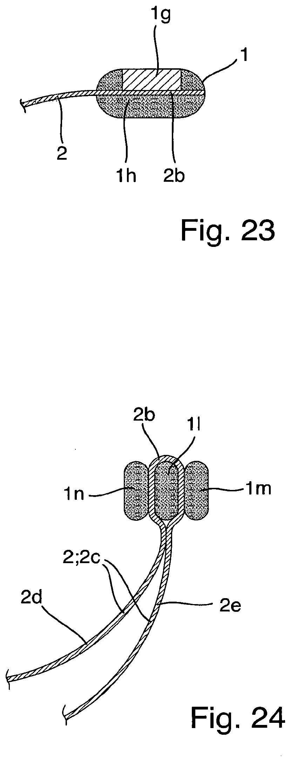

In den Ausführungsbeispielen der Fig. 1 bis 20 ist das Fangnetz jeweils senkrecht zur Schlingenebene in die Schlinge eingefügt, z.B. an einer Unterseite der Schlinge. In alternativen Ausführungen, wie exemplarisch im Ausführungsbeispiel der Fig. 23 gezeigt, ist das Fangnetz 2 parallel zur Schlingenebene in die Schlinge 1 eingefügt. Fig. 23 zeigt dies am Beispiel einer zweikomponentigen Schlingenausführung entsprechend Fig. 20, in alternativen Ausführungen ist die Schlinge analog zu den Ausführungsbeispielen der Fig. 1 bis 19 einkomponentig oder mehrteilig ausgeführt. Bei diesen Ausführungsvarianten mit parallel zur Schlingenebene eingefügtem Fangnetz weist der Querschnitt der Schlinge 1, wie im Fall von Fig. 23, vorzugsweise eine in der Schlingenebene größere Länge als in der dazu senkrechten Ebene auf, im Unterschied beispielsweise zu den Varianten gemäß den Fig. 17 und 20.

Fig. 24 veranschaulicht eine Ausführungsvariante der Erfindung mit drei konformen Schlingenteilen, einem mittleren Schlingenteil 11, einem äußeren Schlingenteil 1m und einem inneren Schlingenteil 1n. Das Fangnetz 2 ist in diesem Beispiel analog zur Ausführungsvariante von Fig. 22 zweilagig mit den Netzlagen 2d und 2e aus dem Schlauchstück 2c gebildet, wobei der Netzhalteabschnitt 2b das mittlere Schlingenteil 1l umschlingt, so dass wiederum beide Netzlagen 2d, 2e an einer gleichen Seite aus der Schlinge 1 herausgeführt sind, vorzugsweise an der Unterseite der Schlinge, von der aus sich das Fangnetz ausgehend von der Schlingenebene nach unten erstreckt. Der Netzhalteabschnitt 2b ist hierbei einerseits zwischen dem mittleren Schlingenteil 1l und dem äußeren Schlingenteil 1m und andererseits zwischen dem mittleren Schlingenteil 1l und dem inneren Schlingenteil 1n gehalten. Die Fixierung der drei Schlingenteile 1l, 1m, 1n und des Netzhalteabschnitts 2b des Fangnetzes 2 kann auf eine der oben zu den anderen Ausführungsbeispielen erläuterten Weise erfolgen, z.B. durch Fixieren aller vier Teile aneinander oder durch Fixieren des Netzhalteabschnitts 2b an einem, zwei oder allen drei Schlingenteilen 1l, 1m, 1n und/oder durch Fixieren der drei Schlingenteile 1l, 1m, 1n jeweils paarweise oder gemeinsam.

Wie die gezeigten und oben erläuterten Ausführungsbeispiele deutlich machen, stellt die Erfindung ein medizinisches Fangnetzschlingeninstrument zur Verfügung, das vergleichsweise einfach hergestellt werden kann und eine sehr hohe Funktions- und Ausfallsicherheit bietet. Insbesondere ist das Fangnetz mit relativ geringem Aufwand sehr sicher und vor unerwünschten Verschiebungen an der Schlinge gehalten.

Es steht versteht sich, dass die Erfindung weitere vorteilhafte Ausführungen umfasst, beispielsweise solche, bei denen die Schlinge aus drei, vier oder mehr konformen, aneinanderliegenden Schlingenteilen oder aus drei, vier oder mehr Schlingenkomponenten in einer herkömmlichen Metall- oder Kunststoffgießtechnik einteilig aufgebaut ist, wobei der Netzaufnahmespalt zwischen wenigstens zwei dieser Schlingenteile bzw. Schlingenkomponenten gebildet ist. Für das Fangnetz sind neben der gezeigten Beutel-/Taschenform beliebige andere, an sich für diesen Einsatzzweck bekannte Fangnetzformen verwendbar.

## Patentansprüche

1. Medizinisches Fangnetzschlingeninstrument mit
- einer distalen Schlinge (1) und
- einem mit einem Netzhalteabschnitt (2b) von der Schlinge im Inneren des Querschnitts der Schlinge gehaltenen Fangnetz (2),
**dadurch gekennzeichnet, dass**
- die Schlinge (1) mindestens zwei konforme Schlingenteile (1a, 1c; 1b, 1d) beinhaltet, die unter Belassung eines Netzaufnahmespaltes (3) dazwischen aneinanderliegend miteinander verbunden sind, und das Fangnetz (2) mit dem Netzhalteabschnitt (2b) im Netzaufnahmespalt festgehalten ist, wobei die mindestens zwei Schlingenteile mittels mindestens einer Klebestellenverbindung auf der vom Fangnetz abgewandten Seite und/oder mittels mindestens einer Klemmstellenverbindung aneinander fixiert sind und/oder ein inneres Schlingenteil (1b) und ein äußeres Schlingenteil (1a) gleichen Querschnitts beinhalten, welches das innere Schlingenteil umgibt und mit einem Innenumfang einem Außenumfang des inneren Schlingenteils zugewandt ist, oder wobei ein erstes Schlingenteil (1c) einen U-förmigen Querschnitt aufweist und ein zweites Schlingenteil (1d) einen rechteckförmigen Querschnitt aufweist und das erste Schlingenteil das zweite Schlingenteil U-förmig auf drei Seiten umgreift.

2. Medizinisches Fangnetzschlingeninstrument mit
- einer distalen Schlinge (1) und
- einem mit einem Netzhalteabschnitt (2b) von der Schlinge im Inneren des Querschnitts der Schlinge gehaltenen Fangnetz (2),
**dadurch gekennzeichnet, dass**
- die Schlinge (1) einteilig unter Einbettung des Netzhalteabschnitts (2b) des Fangnetzes in das Innere des Querschnitts der Schlinge als ein einkomponentiges Gießteil oder mehrkomponentiges Gießteil wenigstens mit einer ersten Schlingenkomponente aus einem ersten Material aus Metall oder Kunststoff und einer daran angegossenen zweiten Schlingenkomponente aus einem vom ersten verschiedenen zweiten Material gefertigt ist und das Fangnetz an seinem Netzhalteabschnitt in dem Gießteil eingegossen gehalten ist.

3. Medizinisches Fangnetzschlingeninstrument mit
- einer distalen Schlinge (1) und
- einem mit einem Netzhalteabschnitt (2b) von der Schlinge im Inneren des Querschnitts der Schlinge gehaltenen Fangnetz (2),
**dadurch gekennzeichnet, dass**
- das Fangnetz (2) von einem umfangsseitig geschlossenen, zusammengelegten Netzgewebe-Schlauchstück (2c) gebildet ist, das sich in Umfangsrichtung durch das Innere des Querschnitts der Schlinge (1) hindurch erstreckt.

4. Medizinisches Fangnetzschlingeninstrument nach Anspruch 1, weiter **dadurch gekennzeichnet, dass** mindestens ein erstes und ein zweites der Schlingenteile mittels mindestens einer Schweiß-/Lötstellenverbindung aneinander fixiert sind.

5. Medizinisches Fangnetzschlingeninstrument nach Anspruch 1 oder 4, weiter **gekennzeichnet durch** eine Klammer (6), die mindestens das erste und das zweite Schlingenteil wenigstens abschnittweise zusammenklemmend umgreift

6. Medizinisches Fangnetzschlingeninstrument nach einem der Ansprüche 1 und 3 bis 5, weiter **dadurch gekennzeichnet, dass** das Fangnetz mittels mindestens einer Klemmstellenverbindung und/oder mindestens einer Klebestellenverbindung und/oder mindestens einer Schweiß-/Lötstellenverbindung an der Schlinge gehalten ist.

7. Medizinisches Fangnetzschlingeninstrument nach einem der Ansprüche 1, 2 und 4 bis 6, weiter **dadurch gekennzeichnet, dass** das Fangnetz von einem Netzgewebebeutel (2a) gebildet ist, der mit einem Beutelrand an der Schlinge festgehalten ist.

8. Medizinisches Fangnetzschlingeninstrument nach einem der Ansprüche 1 und 4 bis 7, weiter **dadurch gekennzeichnet, dass** mindestens ein erstes der Schlingenteile mit dem Netzhalteabschnitt des Fangnetzes und/oder mindestens einem zweiten der Schlingenteile mittels einer Klebestellenverbindung verbunden ist, die sich ausschließlich an einer oberen Seite der Schlinge befindet, wobei sich das Fangnetz nur an einer gegenüberliegenden unteren Seite in die Schlinge hinein erstreckt.

## Claims

1. Medical net-and-loop type retrieval instrument comprising
- a distal loop (1) and
- a retrieval net (2) that by way of a net holding portion (2b) is firmly held by the loop (1) in the interior of the cross section of the loop,
**characterized in that**
- the loop comprises at least two conforming loop parts (1a, 1c; 1b, 1d) which while leaving a net receptacle gap (3) therebetween are connected so as to bear on one another, and the retrieval net (2) by way of the net holding portion (2b) is firmly held in the net receptacle gap, wherein the at least two loop parts are fixed to one another by means of at least one adhesively bonded joint connection on the side opposite from the retrieval net and/or by means of at least one clamped joint connection and/or comprise an inner loop part (1b) and an outer loop part (1a) of equal cross-section which surrounds the inner loop part and faces with an inner periphery an outer periphery of the inner loop part, or wherein a first loop part (1c) comprises a U-shaped cross-section and a second loop part comprises a rectangular cross-section and the first loop part encompasses the second loop part in a U-shape on three sides.

2. Medical net-and-loop type retrieval instrument comprising
- a distal loop (1) and
- a retrieval net (2) that by way of a net holding portion (2b) is firmly held by the loop (1) in the interior of the cross section of the loop,
**characterized in that**
- the loop (1) is made integrally, while embedding the net holding portion (2b) of the retrieval net in the interior of the cross section of the loop, as a single-component casting or a multi-component casting having at least one first loop component from a first material from metal or plastics material, and a second loop component, molded to said first loop component, from a second material that is different from the first material, and the retrieval net at the net holding portion thereof is held so as to be cast in the casting.

3. Medical net-and-loop type retrieval instrument comprising
- a distal loop (1) and
- a retrieval net (2) that by way of a net holding portion (2b) is firmly held by the loop (1) in the interior of the cross section of the loop,
**characterized in that**
- the retrieval net (2) is formed by a folded-together woven-fabric net hose piece (2c) which is circumferentially closed and which in the circumferential direction extends through the interior of the cross section of the loop (1).

4. Medical net-and-loop type retrieval instrument as claimed in claim 1, further **characterized in that** at least a first and a second of the loop parts are fixed to one another by means of at least one welded/soldered joint connection.

5. Medical net-and-loop type retrieval instrument as claimed in claim 1 or 4, further **characterized by** a clip (6) which at least in portions encompasses at least the first and the second loop part so as to conjointly clamp them together.

6. Medical net-and-loop type retrieval instrument as claimed in any one of claims 1 and 3 to 5, further **characterized in that** the retrieval net is held on the loop by means of at least one clamped joint connection and/or at least one adhesively bonded joint connection and/or at least one welded/soldered joint connection.

7. Medical net-and-loop type retrieval instrument as claimed in any one of claims 1, 2, and 4 to 6, further **characterized in that** the retrieval net is formed by a woven-fabric net pouch (2a) which by way of a pouch periphery is firmly held on the loop.

8. Medical net-and-loop type retrieval instrument as claimed in any one of claims 1 and 4 to 7, further **characterized in that** at least a first one of the loop parts by means of an adhesively bonded joint connection is connected to the net holding portion of the retrieval net and/or to at least a second one of the loop parts, said adhesively bonded joint connection being situated exclusively on an upper side of the loop, wherein the retrieval net extends into the loop only on an opposite lower side.

## Revendications

1. Instrument médical avec anse à filet de retenue, avec
- une anse distale (1) et
- un filet de retenue (2) tenu à l'intérieur de la section de l'anse par l'anse utilisant une section de maintien du filet (2b),
**caractérisé en ce que**
- l'anse (1) contient au moins deux parties d'anse (1a, 1c ; 1b, 1d) conformes qui sont reliées ensemble de manière adjacente en laissant une fente de logement du filet (3) entre elles, et que le filet de retenue (2) est maintenu en place dans la fente de logement de filet par la section de maintien de filet (2b), sachant que lesdites au moins deux parties d'anse sont fixées l'une à l'autre au moyen d'au moins un assemblage par points de colle sur le côté opposé du filet de retenue et/ou au moyen d'au moins un assemblage par points de pincement et/ou contiennent une partie d'anse intérieure (1b) et une partie d'anse extérieure (1a) de la même section qui entoure la partie d'anse intérieure et est tournée au niveau d'une circonférence intérieure vers une circonférence extérieure de la partie d'anse intérieure, ou sachant qu'une première partie d'anse (1c) présente une section en forme de U et qu'une seconde partie d'anse (1d) présente une section rectangulaire et que la première partie d'anse enveloppe la seconde partie d'anse de manière à former un U sur trois côtés.

2. Instrument médical avec anse à filet de retenue, avec
- une anse distale (1) et
- un filet de retenue (2) tenu à l'intérieur de la section de l'anse par l'anse utilisant une section de maintien du filet (2b),
**caractérisé en ce que**
- l'anse (1) est fabriquée en un élément en intégrant la section de maintien (2b) du filet de retenue à l'intérieur de la section de l'anse, sous forme de pièce moulée monocomposant ou de pièce moulée multicomposant au moins avec un premier composant d'anse constitué d'un premier matériau en métal ou plastique et un second composant d'anse surmoulé, constitué d'un second matériau différent du premier et que le filet de retenue est maintenu sur sa section de maintien de filet, coulé dans la pièce moulée

3. Instrument médical avec anse à filet de retenue, avec
- une anse distale (1) et
- un filet de retenue (2) tenu à l'intérieur de la section de l'anse par l'anse utilisant une section de maintien du filet (2b),
**caractérisé en ce que**
- le filet de retenue (2) est constitué d'une portion de tube (2c) en résille regroupée, fermée du côté circonférentiel, qui s'étend à travers l'intérieur de la section de l'anse (1) dans le sens circonférentiel.

4. Instrument médical avec anse à filet de retenue selon la revendication 1, caractérisé également en ce qu'au moins une première partie d'anse et une seconde sont fixées l'une à l'autre au moyen d'au moins un assemblage par points de soudure/brasure.

5. Instrument médical avec anse à filet de retenue selon la revendication 1 ou 4, caractérisé également en une pince (6) qui enveloppe au moins la première et la seconde parties d'anse, au moins partiellement en les pinçant ensemble.

6. Instrument médical avec anse à filet de retenue selon l'une des revendications 1 et 3 à 5, caractérisé également en ce que le filet de retenue est maintenu à l'anse au moyen d'au moins un assemblage par points de pincement et/ou au moins un assemblage par points de colle et/ou au moins un assemblage par points de soudure/brasure.

7. Instrument médical avec anse à filet de retenue selon l'une des revendications 1, 2 et 4 à 6, caractérisé également en ce que le filet de retenue est constitué d'une poche en résille (2a) qui est fixée à l'anse par un bord de la poche.

8. Instrument médical avec anse à filet de retenue selon l'une des revendications 1 et 4 à 7, caractérisé également en ce qu'au moins une première des parties d'anse est reliée à la section de maintien du filet de retenue et/ou au moins à une deuxième des parties d'anse au moyen d'un assemblage par points de colle qui se trouve uniquement sur une partie supérieure de l'anse, sachant que le filet de retenue ne s'étend dans l'anse que sur une partie inférieure opposée.
